# EUROPEAN PATENT APPLICATION

(11) **EP 1 647 283 A1**
(43) Date of publication of application: **19.04.2006**
(21) Application number: 04024313.1
(22) Date of filing: 12.10.2004
(51) Int. Cl.: A61K 49/12

(54) **Polymeric contrast agent**

(71) Applicant: Deutsches Krebsforschungszentrum Stiftung des öffentlichen Rechts, 69120 Heidelberg (DE)
(72) Inventor: Kiessling, Fabian, Dr., 69121 Heidelberg (DE); Heilmann, Melanie, 63474 Bad Neuenahr-Ahrweiler (DE); Semmler, Wolhard, Prof. Dr., 69118 Heidelberg (DE); Debus, Jörg, Prof. Dr., 69121 Heidelberg (DE); Peschke, Peter, Dr., 69251 Gaiberg (DE); Ulrich, Karel, 140 00 Praha 4 (CZ); Subr, Fladimir, 1643 Melnik (CZ)
(74) Representative: Huhn, Michael

(57) **Abstract**

The present invention relates to a contrast agent comprising a copolymer having at least a first monomer unit which comprises a metal chelating moiety and a second monomer unit without a chelating moiety and a contrast enhancing metal bound to the chelating moiety.

The invention also relates to a process for the preparation of such a contrast agent and the use in magnetic resonance imaging (MRI).

## Description

The present invention relates to a contrast agent comprising a copolymer as well as a process for the preparation thereof and the use of such a contrast agent in magnetic resonance imaging.

Magnetic resonance imaging (MRI) is well known in medical diagnostics. In a strong magnetic field radio-frequency (rf) pulses are used to excite free protons in tissue. After rf excitation relaxation of the magnetization occurs in two different ways. Depending on tissue properties those two effects are described by the time constants, longitudinal (T₁) and transversal (T₂) relaxation time. Usually liquid parts of the tissue are hyperintense in T₂-weighted MR images and hypointense in T₁-weighted MR images. Fatty tissue is hyperintense in both methods. Due to local edema pathologies are often better assessed by T₂-weighted techniques. In contrast, T₂-weighted imaging is higher sensitive to susceptibility artefacts which can occur due to high local blood flow or tissue bleeding. Thus, the morphological assessment is often better on the T₁-weighted images.

To improve the sensitivity and/or specificity of the T₁-weighted imaging technique the application of a contrast agent is advantageous. In MRI normally a paramagnetic metal is used as contrast agent. However, the toxic effects of such a metal have to be avoided. Therefore, such metals are applied in form of a complex with chelating organic ligands. Most commonly used are small chelates of gadolinium, mostly as complex with diethylenetriamine pentaacetic acid (DTPA). They are marked by a fast renal clearance, early extravasation and a low toxicity. This makes them suitable for many clinical implementations such as the detection and delineation of pathologically altered tissue or micro-angiographies of the large circulation. However, the fast extravasation is disadvantageous and often prevents successful functional experimentation on tissue vascularization. Additionally, the visualization of micro-vessels is complicated because long scanning times are required to achieve a suitable signal to noise ratio and these are not compatible with the kinetics of these contrast agents.

Therefore, numerous attempts have been made to design contrast agents with larger molecular size in order to circumvent these limitations.

US 6, 270,748 describes an image enhancing agent having a polymeric core including an image enhancing compound, i.e. the metal which is chemically bound to the core, and a polymeric shell surrounding the core with the compound. The core is formed from an acid monomer and an esterified monomer. The acid monomer is e.g. (meth)acrylic acid and the esterified monomer may be a (meth)acrylic acid ester. The gadolinium is chemically bound by complexation with several carboxylic functions of various monomers forming the copolymer. This approach is disadvantageous because the gadolinium is not bound by a distinct complexation moiety of a monomer and therefore, the directed introduction of the metal is not possible. Furthermore, the shell which does not contain the metal like gadolinium prevents the interaction with the protons of the surrounding at a certain degree that may result in lower relaxation and sensitivity.

Other attempts were made based on natural polymers like proteins, especially human serum albumin (HSA). Here, a suitable ligand is chemically connected with the protein and subsequently the contrast enhancing agent is introduced. This is disadvantageous because of the limited control where the ligand and the contrast enhancing agent are located within the protein. Moreover, such systems could be immunogenic,

Another alternative are dendrimeric contrast agents with medium molecular size like Gadomer 17 but these show only moderate intravasation half-life of several minutes and they have mostly renal excretion (Kobayashi H. et al., Mol. Imaging 2003 (2), 1-10).

Thus, the object of the present invention is to provide an improved polymer-based contrast agent having good selectivity and relaxation values. Chelating of the contrast enhancing agent with a synthetic polymer results in decreased extravasation, prolonged half-life in circulation and increased accumulation in solid tumours.

Accordingly, the present invention provides a contrast agent comprising:
a) a copolymer having at least a first monomer unit, which comprises a metal chelating moiety and a second monomer unit without a chelating moiety; and
b) a contrast enhancing metal bound to the chelating moiety.

It was found, that the introduction of the chelating moiety at the "monomer unit level" results, after the copolymer complexation with the metal, in a polymeric contrast agent where e.g. the relaxation time may be improved.

This may be caused by the fact that there is control over the concentration of the chelating moiety within the polymer. This is not given e.g. by a protein where the reaction with the chelating molecule may occur at preferred regions of the protein which are not necessarily homogenously present in such a protein. This is clear when the reaction only takes place with certain amino acids which are not homogenously represented in the amino acid sequence of the protein.

According to the present invention, it is clear that the chemical structure of the monomer units correspond to the monomers used for the formation of the copolymer. Further, the structure of the monomers may differ because of optionally present protective groups which may be needed in the polymerization step (co-polymer formation step). These protective groups are usually not considered when discussing the structure of a monomer unit because they will be removed in any case after the critical synthesis step, i.e. the polymerization step.

Preferably, the copolymer is water-soluble and the second monomer is hydrophilic.

Preferably, the copolymer has a molecular weight within the range of 5 kDa to 200 kDa.

It is advantageous when the copolymer is molecularly dissolved in water and has a random-coil or expanded structure in solution. The term random coil originates from a simple model of a structure of a polymer chain in a solution. Here, the direction of the chain bond at each repeating unit is random so that the shape of the polymer chain can be visualized through the random walk model and its appearance looks like a coil.

Suitable monomers, especially for preparation of a water-soluble polymer, are acrylic and methacrylic acid, acrylamide and methacrylamide, selected acrylates, methacrylates, N-substituted acrylamides and methacrylamides, *N*-vinylpyrrolidone and their derivatives.

Preferably the second monomer unit is selected from the group consisting of N-vinylpyrrolidone, acrylic and methacrylic acid, acrylamid and methacrylamide, alkyl acrylate, alkyl methacrylate, alkyl acrylamide and alkyl methacrylamide, wherein alkyl consists of 1 to 6 carbons, (C₁₋₆ alkyl), which is linear or branched and optionally substituted with one or more substituents independently selected from the group consisting of; -OH, -N(R¹)₂ and -N(R¹)₃⁺ (together with a suitable anion), wherein each R¹ is independently selected from the group consisting of H and C₁₋₃ alkyl.

Preferred the alkyl is C₁₋₆ alkyl which is linear or branched and optionally substituted with at least on of the groups consisting of -OH, -NH(CH₃), -N(CH₃)₂ and -N(CH₃)₃⁺.

Even more preferred is alkyl selected from the group consisting of methyl, ethyl, dimethylaminoethyl, hydroxyethyl and hydroxypropyl groups.

In a most preferred embodiment the second monomer is *N*-(2-hydroxypropy)methacrylamide (HPMA).

Copolymers bearing chelating groups can be prepared by the radical solution or precipitation copolymerization of the above specified monomers with monomers containing chelating groups.

Within the meaning of the present invention the term C₁₋₂₀ alkyl means an alkyl chain having 1 to 20 carbon atoms, e.g. C₁₋₆-alkyl, methyl, ethyl, n-propyl, isopropyl, n-butyl, isobutyl, sec-butyl, tert-butyl, pentyl, hexyl, heptyl, octyl, nonyl, decanyl, undecanyl dodecanyl, tetradecayl, pentadecanyl, hexadecanyl, heptadecanyl, octadecanyl, nonadecanyl, eicosanyl. Each hydrogen of a C₁₋₂₀-alkyl carbon may be replaced by a substituent.

C₁₋₆ alkyl means an alkyl chain having 1 to 6 carbon atoms, e.g. methyl, ethyl, n-propyl, isopropyl, n-butyl, isobutyl, sec-butyl, tert-butyl, n-pentyl or hexyl. Each hydrogen of a C ₁₋₆ alkyl carbon may be replaced by a substituent.

C₁₋₄ alkyl means methyl, ethyl, n-propyl, isopropyl, n-butyl, isobutyl, sec-butyl or tert-butyl.

C₁₋₃ alkyl means methyl, ethyl, n-propyl or isopropyl.

Preferably, the first monomer unit is selected from the group consisting of alkyl acrylate, alkyl methacrylate, alkyl acrylamide and alkyl methacrylamide, wherein alkyl is C₁₋₂₀ - alkyl which is linear or branched and which is optionally interrupted by one or more atoms or atom groups independently selected from the group consisting of -O-, -N(R²)-, -C(O)-O-, and -C(O)-N(R²)-. Optionally C₂₋₂₀ alkyl is substituted with one or more substituents independently selected from the group consisting of -OH, and -N(R²)₂, wherein each R² is independently selected from the group consisting of H; and C₁₋₄ alkyl and wherein the chelating moiety is part of the C₁₋₂₀ alkyl chain and said chelating moiety comprises at least two carboxylic or carboxylate groups.

Preferably, the chelating moiety comprises one or more amino polycarboxylic acids.

Preferred acids are independently selected from the group consisting of ethylenediamine tetraacetic acid (EDTA), DTPA, aspartic acid and glutamic acid.

The contrast agent according to the present invention has preferably a first monomer unit which is represented by formula (I): 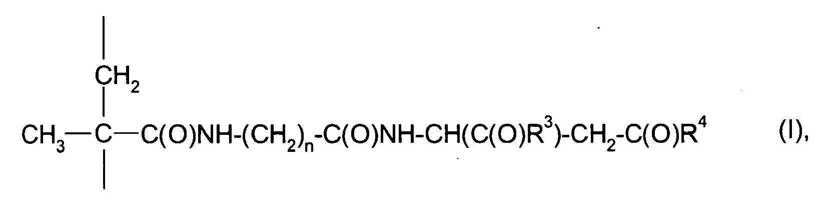 wherein n is an integer from 1 to 10 and R³, R⁴ are independently selected from the group consisting of OH, and -NH-CH(COOH)-CH₂-COOH).

The advantage of the contrast agent according to the present invention is that the property of the contrast agent and the concentration of the contrast enhancing agent can easily be adapted by varying the molar ratio in the first and second monomer units in a copolymer.

Preferably, the molar ratio of first monomer : second monomer unit is from 0,005 to 0,3.

The contrast enhancing metal is preferably a paramagnetic metal ion.

More preferred are metal ions selected from the group consisting of gadolinium, manganese, copper, chromium, iron, cobalt, erbium, nickel, europium and dysprosium.

Most preferred is gadolinium.

A most preferred embodiment of a contrast agent comprises a copolymer of the formula (II) consisting of HPMA (second monomer) and HPMA derivative (first monomer) 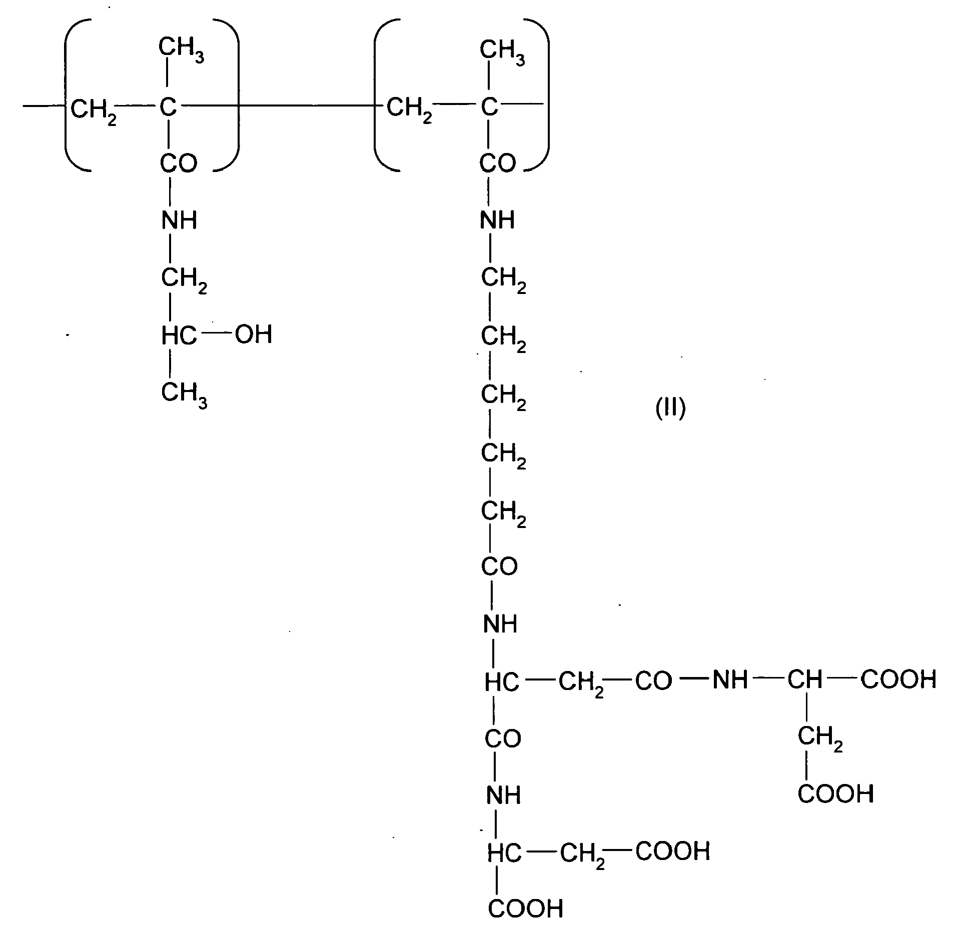 containing various amounts of the first monomer unit, preferably from 0.5 to 30 mol-%.

Another object of the present invention is a process for the preparation of a contrast agent according to the present invention.

This process comprises the steps of:
a) polymerizing the first monomer with at least a second monomer in solution, wherein functional groups of the monomer are optionally protected;
b) optionally de-protecting the formed copolymer;
c) adding the contrast enhancing metal as a salt to a copolymer solution; and
d) isolating the metal copolymer complex.

Preferably, the polymer formation step a) is a radical solution (homogeneous) or precipitation (heterogeneous) copolymerization. Optionally functional groups, especially carboxylic groups comprised by the chelating moiety, are protected. A suitable protective group is an alkyl ester, preferably a methyl ester or t.-butyl ester.

The formed copolymer contrast enhancing metal complex may be isolated by dialysis and subsequent lyophilization.

Another object of the invention is the use of a contrast agent according to the present invention in magnetic resonance imaging (MRI), especially in MR angiography.

The following examples shall illustrate the invention without limiting the scope of the invention.

### Examples

### Example 1: Synthesis of monomers

***N*-(2-Hydroxypropyl)methacrylamide** (HPMA) is synthesized as described earlier [ K. Ulbrich, V. Subr, J. Strohalm, D. Plocová, M. Jelínková, B. Ríhová, Polymeric drugs based on conjugates of synthetic and natural macromolecules I. Synthesis and physico-chemical characterisation. J. Controlled Release 64 (2000) 63-79.]. M.p. 70°C, elemental analysis: Calcd/found C 58.80/58.98, H 9.16/9.18, N9.79/9.82

### Synthesis of Ma-Acap-Asp-[(Asp-(OMe)₂]₂

This monomer is used for preparation of the copolymer carrier via copolymerisation with HPMA. It was prepared by coupling of Ma-Acap-OH (for synthesis see below) with a derivative of aspartic acid Asp-[Asp-(OMe)₂]₂.

### Synthesis of derivative of aspartic acid

### Synthesis of N-tert-butyloxycarbonyl-L-aspartic acid, Boc-Asp-OH (A)

L-aspartic acid (10 g, 0.075 mol) and sodium hydroxide (6.0 g, 0.15 mol) are dissolved in distilled water (100 mL). The solution is cooled in ice bath to 0°C and dioxane (100 mL) was added. (Boc)₂O (18.0 g, 0.083 mol) is added dropwise during 1 h. Reaction mixture is stirred in ice bath for 2 hours and then another 2 h at room temperature. Reaction mixture is concentrated on vacuum evaporator. The unreacted (Boc)₂O is removed by extraction into the diethyl ether. The aqueous layer is separated, acidified to pH=2 with saturated solution of sodium hydrogen sulfate and the product is extracted into the ethyl acetate. Organic phase is separated, dried with sodium sulfate and concentrated on vacuum evaporator. Boc-L-Asp-OH was crystallized from mixture ethyl acetate/hexane. Yield: 13.4 g (76.5%), m.p. 116-118°C.

### Synthesis of L-Aspartic acid bis methylester hydrochloride HCl.Asp-(OMe)₂ (B)

Methanol (100 mL), dried with calcium hydride, is put into 250 mL two neck round bottom flask equipped with stirrer and condenser. Methanol is cooled to -10°C and the thionyl chloride (16.4 mL, 0.225 mol) is added dropwise during 30 min. L-Aspartic acid (15.0 g, 0.112 mol) is added and a reaction mixture was stirred and refluxed for 5 h. The reaction mixture is cooled to room temperature and concentrated on vacuum evaporator. The final product is crystallized from a mixture methanol/diethyl ether. Yield: 18.3 g (82.5%), m.p. 115-117°C.

### Synthesis of TFA.Asp-[Asp-(OMe)₂]2 (C)

Boc-Asp-OH (1.475 g, 6.33 mmol), HCl.Asp-(OMe)₂ (2.5 g, 12.66 mmol) and Et₃N (1.77 mL, 6.33 mmol) are mixed in 50 mL of tetrahydrofurane (THF) and suspension is cooled to -10°C. The DCC (3.12 g, 15.1 mmol) is dissolved in 20 mL THF and the solution is cooled to -10°C. Cool solution of DCC in THF is added to the suspension and the reaction mixture is left one hour at -10°C and then overnight at 5°C without mixing. Reaction mixture is warmed up to room temperature, acetic acid (0.1 mL) is added and a mixture is stirred for 30 min at room temperature. Precipitated *N,N*'-dicyclohexylurea (DCU) and triethylamine hydrochloride (Et₃N.HC1) are filtered off, and a filtrate is concentrated on vacuum evaporator. Oily residue is diluted with ethyl acetate (100 mL) and gradually extracted with 2 wt% aqueous sodium hydrogen carbonate (50 mL), 0.5 wt% citric acid (50 mL) and water (50 mL) in a last step. Organic layer is dried with sodium sulfate and product is crystallized from a mixture ethyl acetate/hexane. Yield: 2.1g (64.5%), m.p. 115-116°C, TLC: (silica gel, ethyl acetate, R_{f} = 0.54). Elemental analysis: Calcd/found C = 48.55/48.85, H = 6.40/6.50, N = 8.09/8.05. The Boc protection group is removed by trifluoroacetic acid (TFA). Boc-Asp-[Asp-(OMe)₂]2 (2.0 g) is dissolved in TFA (8.0 mL) and stirred for 30 min at room temperature. The excess of TFA is evaporated, the mixture was tree times diluted with dry methanol and the solvent evaporated again. The oilly residue is triturated with diethyl ether to form crystals of TFA.Asp-[Asp-(OMe)₂]₂. Yield 1.6 g (77.9%)

### Synthesis of N-methacryloyl-6-amino hexanoic acid, Ma-Acap-OH (D)

The Schotten-Baumann acylation method with methacryloyl chloride in an aqueous alkaline medium is used for methacryloylation of 6-aminohexanoic acid [J.Drobnik, J.Kopecek, J.Labský, P.Rejmanová, J.Exner, V.Saudek, J.Kálal, Enzymatic cleavage of side chains of synthetic water-soluble polymers, Makromol. Chem. 177 (1976) 2833-2848]. (m.p. 52-53 °C, elemental analysis: Calcd/found C 60.28/60.43, H 8.60/8.37, N 7.03/6.98)

### Synthesis of Ma-Acap-Asp-[(Asp-(OMe)₂]₂ (E)

*N*-methacryloyl 6-aminohexanoic acid (0.5 g, 2.51 mmol) and Et₃N (0.385 mL, 2.76 mmol) are dissolved in THF (5mL). The solution is cooled to -10 °C and isobutyl chloroformate (0.36 mL, 2.76 mmol) was added. After 10 min this, the reaction mixture is added to a cooled mixture of TFA.Asp-[Asp-(OMe)₂]₂ (1.47 g, 2.76 mmol) and Et₃N (0.424 mL,3.04 mmol) in THF (5 mL). Reaction mixture is stirred for 4 h at room temperature. The precipitated Et₃N.HC1 is filtered off and THF is evaporated in vacuum. Residue is dissolved in ethyl acetate (20 mL) and gradually extracted with 2 wt% aqueous sodium hydrogen carbonate (5 mL), 0.5 wt% citric acid (5 mL) and water (5 mL). Organic layer is dried with sodium sulfate and the product is crystallized from a mixture ethyl acetate/hexane. Yield 0.5g (33 %), m.p. 110-113 °C, elemental analysis: Calcd/found C = 51.99/50.31, H = 6.71/6.34, N = 9.33/8.93.

Optionally, the monomer Ma-Acap-Asp-[(Asp-(OMe)₂]₂ could be prepared by the reaction of N-methacryloyl-6-aminohexanoic acid thiazolidine-2-thione with TFA.Asp-[Asp-(OMe)₂]₂ as it is shown below:

### Synthesis of N-methacryloyl-6-aminohexanoic acid thiazolidine-2-thione

*N*-methacryloyl-6-aminohexanoic acid (3.0 g, 0.015 mol) and 2-thiazoline-2-thiol (1.8g, 0.015 mol) are dissolved in 35 mL of tetrahydrofurane (THF). DCC (3.72g, 0.018 mol) is dissolved in THF (5 mL). Both solutions were cooled down to -15 °C. The cooled solutions are mixed and kept for 1 hour at -15 °C and then overnight at 5°C. The reaction mixture is stirred for 1 hour at room temperature, and then 0.1 mL of acetic acid is added. Mixture is stirred for 1 hour. The precipitated *N*,*N*'-dicyclohexylurea (DCU) is filtered off. The solution is evaporated in vacuum and diluted with ethyl acetate. The second part of DCU is removed by filtration. The solution is evaporated in vacuum and diethyl ether is added and product crystallised at ―15 °C. Product was filtered off, washed with diethyl ether and dried in vacuum. Yield : 3.56 g (78%). (m.p. 61-62 °C, elemental analysis: Calcd/found C 51.98/52.03, H 6.72/6.53, N 9.33/9.34, S 21.35/21.10)

### Synthesis of Ma-Acap-Asp-[(Asp-(OMe)₂]₂ from N-methacryloyl-6-aminohexanoic acid thiazolidine-2-thione

*N*-methacryloyl-6-aminohexanoic acid thiazolidine-2-thione (0.5 g, 1.6 mmol) and TFA.Asp-[Asp-(OMe)₂]₂ (0.977 g, 1.83 mmol) is dissolved in ethyl acetate (3 ml). Triethylamine (0.281 ml, 0.002 mmol) is added dropwise during 15 min. Reaction mixture is stirred 6 h at room temperature. The product, Ma-Acap-Asp-[(Asp-(OMe)₂]₂, precipitates from the reaction mixture. Product is filtered off, washed with ethylacetate and dried in vacuum. Crude product (0.7 g) is recrystalized from the methanol diethyl ether mixture. Yield: 0.58 g (58%). (m.p. 144-146 °C, elemental analysis: Calcd/found C 51.99/51.96, H 6.71/6.71, N 9.33/9.20)

### Example 2: Synthesis of the HPMA - Ma-Acap-Asp-[(Asp-(OMe)₂]₂ copolymer (F)

The copolymer is prepared by solution radical copolymerization of HPMA with Ma-Acap-Asp-[(Asp-(OMe)₂]₂ in methanol at 60 °C. HPMA (1.0 g, 6.98 mmol) and Ma-Acap-Asp-[(Asp-(OMe)₂]₂ (0.221 g, 0.368 mmol) are dissolved in methanol (7.0 mL) and initiator AIBN (0.068 g) is added. Solution was introduced into glass ampoule and bubbled with nitrogen. The ampoule is sealed and polymerization is carried out at 60 °C for 24 h. The polymer is isolated by precipitation into a mixture of acetone and diethyl ether (3: 1), filtered off, washed with acetone, diethyl ether and dried in vacuum. Yield: 0.998 g (81.7%). Molecular weight of copolymer (M_{w} 24 800, M_{w}/Mₙ 1.92) is determined by size exclusion chromatography on HPLC system AKTA explorer (Pharmacia) equipped with RI and multiangle light scattering detectors DAWN DSP-F (Wyatt Technol. Corp., USA) on Superose 6 column. Copolymers differing in chemical composition and molecular weight can be easily prepared by changing ratio of monomers (HPMA : Ma-Acap-Asp-[(Asp-(OMe)₂]₂), content of initiator and concentration of monomers in the polymerisation mixture. Polymers with a content of of Ma-Acap-Asp-[(Asp-(OMe)₂]₂ up to 30 % and molecular weights 200 000 g/mol can be prepared.

The methyl ester groups are removed by alkaline hydrolysis. Briefly, copolymer (0.8 g) is dissolved in distilled water and pH of the solution is maintained at 11.5 with addition of 0.1 M NaOH for 6 hours at room temperature. Then pH is lowered to 2 with diluted hydrochloric acid and a copolymer is dialyzed in visking dialyse tube with cut off 3 500 for two days and lyophilized. Yield: 0.75 g.

### Example 3: Synthesis of polymer gadolinium complex (G)

Copolymer P-Acap-Asp-[Asp-(OH)₂]₂ (0.45 g) is dissolved in distilled water and gadolinium chloride (0.045 g) was added. The pH of the solution is gradually increased to 6.0 by addition of 0.1 M NaOH during one hour. The excess of gadolinium is removed from complex by dialysis using visking dialysis tube with cut off 3500 for 48 hours in water. The polymer-gadolinium complex is lyophilized. The yield was 0.41 g of Gd complex.

### Example 4: Characterization of the polymer-Gd complex (HE-45)

The gadolinium content in the polymer complex is determined by atomic mass spectroscopy and its content in copolymer HE-45 was 5.19±0.28 wt%.

### Toxicity of HE-45

The toxicity of the polymer-Gd complex (HE-45) is investigated in-vitro on human fibroblasts using trypan-blue staining. Even at high concentrations and long incubation time no toxicity of HE-45 (as compared with the untreated control cells and cells incubated with Gd-DTPA) is observed.

### Measurement of longitudinal and transversal relaxation time and relaxivity

The longitudinal (T₁) and transversal (T₂) relaxation time are measured for different concentrations of Gd at a 1.5 T MR scanner (Magnetom Vision, Siemens, Erlangen). The T₁-measurement is performed using a saturation-recovery TurboFLASH sequence (TR/TE/α= 8,2ms / 3,9ms / 5°) applying recovery times between 50 ms and 9 s. T₂ determination is done with a spinecho sequence (TR = 2000 ms) aquiring 16 echos at echo times between 23 ms and 360 ms.
For Gd concentrations between 0.016 mmol/L and 0.650 mmol/L T₁-times range between (1859±14)ms and (5.6±12.5)ms and T₂-times between (1502±170)ms and (62±2)ms. The longitudinal relaxivity was r₁ = (18.9±0.1) mM⁻¹s⁻¹ and the transversal r₂ = (26.1±2.7) mM⁻¹s⁻¹. The relaxivities r₁ and r₂ are calculated as means of two measurements being performed with a two-month time slab using the same phantom. Additionally, the longitudinal relaxivity of HE-45 is measured at two different field strengths: r₁ = (18.9±0.1) mM⁻¹ s⁻¹ at 1.5 T and r₁ = (14.0±0.1) mM⁻¹ s⁻¹ at 7.0 T.
MR angiography at 1.5 and 7 T
The impact of HE-45 for MR angiography is investigated at a 1.5 T MR scanner (Symphony, Siemens, Erlangen) by using the above mentioned animal coil. The scan protocol includes a native and a contrast-enhanced measurement of a rat abdomen and head using a FLASH-3D gradient echo sequence (TR/TE=13.3/6.2ms, flip-angle=50°, BW=150 Hz/pixel, NEX=1, FOV=74x85mm, matrix=358x512, voxel-size=166x206x320µm³). Further measurements are performed in nude mice at a high field MR scanner (7 T Pharmascan, Bruker, Ettlingen) in comparison with Gadomer-17 (Schering, Berlin). The pelvis of the animals is examined. Already at 1.5 T an excellent display of abdominal blood vessels of the rat was reached down to a subsegmental level. Also cerebral blood vessels are represented with high contrast and detail. At 7 T the contrast agent shows its superiority as compared with Gadomer-17 concerning the contrast and the display of small vessels.
Dynamic contrast-enhanced magnetic resonance imaging (DCE MRI) at 1.5 T
Two tumor-bearing nude mice are examined by DCE MRI at a 1.5 T MR scanner (Magnetom Vision, Siemens, Erlangen) using a home-built animal coil for excitation and receiption (diameter: 83 mm, length: 120 mm). Using a gradient echo sequence (FLASH: TR/TE/α = 120ms / 6.5ms / 90°) [17] 60 measurements are performed at a temporal resolution of 17 s. At the beginning of the 4^{th} measurement the contrast agent is injected over an infusion time of 5 s via a catheter into the tail vein of both mice. The dose is 0.1 mmol/kg Gadomer-17 for mouse 1 and 0.1 mmol/kg HE-45 for mouse 2. Afterwards, data are post-processed using a pharmacokinetic model [18]. Color-coded parameter maps were calculated and overlaid to the morphological images. The model parameters are amplitude (A) which goes linear with the signal enhancement normalized to the pre-contrast intensity and the exchange rate constant (kₑₚ) which reflects the velocity of contrast agent exchange between capillary and interstitial space.
Gadomer-17 shows in liver a faster signal increase up to 180 % of the pre-contrast value and a following slow decrease of signal down to 125 % of pre-contrast value after 15 min. The model parameters calculated in the liver are: A=0.9 und kₑₚ = 4.2 min⁻¹. With HE-45 the increase in signal intensity occurred immediately after the contrast agent injection and also reaches a maximal value of 180 % in respect on the pre-contrast value. However the decrease of signal intensity is slower as compared to Gadomer-17 and goes down to 160 % of the pre-contrast value after 15 min.
The model parameters of HE-45 in the liver ae: A=0.7 und kₑₚ = 14.0 min⁻¹. While Gadomer-17 extravasated in the whole tumor the early accumulation of HE-45 only is observed at the tumor rim. The determination of the model parameters in a "region of interest" (ROI) covering whole tumor yields A=0.9 and kₑₚ = 0.5 min⁻¹ for Gadomer-17 and A=0.2 und kₑₚ = 0.4 min⁻¹ for HE-45.

## Claims

1. A contrast agent comprising
a) a copolymer, having at least a first monomer unit, which comprises a metal chelating moiety and a second monomer unit without a chelating moiety; and
b) a contrast enhancing metal bound to the chelating moiety.

2. A contrast agent according to claim 1, wherein the copolymer is water-soluble and the second monomer is hydrophilic.

3. The contrast agent according to claim 1 or 2, wherein the copolymer has a molecular weight within the range of 5 kDa to 200 kDa.

4. The contrast agent according to any of the preceding claims, wherein the copolymer is a random-coil polymer in aqueous solution.

5. The contrast agent according to any of the preceding claims, wherein the first and second monomer units are independently selected from the group consisting of acrylic acid, methacrylic acid, acrylate; methacrylate; acrylamide; methacrylamide; N-substituted acrylamide; N-substituted methacrylamide; N-vinylpyrrolidone; and their derivatives.

6. The contrast agent according to any of the preceding claims, wherein the second monomer unit is selected from the group consisting of *N*-vinylpyrrolidone, acrylic and methacrylic acid, acrylamide and methacrylamide, alkyl acrylate; alkyl methacrylate; alkyl acrylamide and alkyl methacrylamide; wherein alkyl is C₁₋₆ alkyl, which is linear or branched and optionally substituted with one or more substituents independently selected from the group consisting of, -OH; -N(R¹)₂; and -N(R¹)₃⁺ wherein each R¹ is independently selected from the group consisting of H; and C₁₋₃ alkyl.

7. The contrast agent according to claim 6, wherein alkyl is C₁₋₆ alkyl which is linear or branched and optionally substituted with at least one of the groups consisting of-OH, -NH(CH₃), -N(CH₃)₂ and -N(CH₃)₃⁺.

8. The contrast agent according to claim 6, wherein alkyl is selected from the group consisting of methyl, ethyl, dimethylaminoethyl, hydroxyethyl and hydroxypropyl.

9. The contrast agent according to claim 8, wherein the second monomer is N-(2-hydroxypropyl)methacryl amide.

10. The contrast agent according to any of the preceding claims, wherein the first monomer unit is selected from the group consisting of alkyl acrylate, alkyl methacrylate, alkyl acrylamide and alkyl methacrylamide, wherein alkyl is C₁₋₂₀ alkyl, which is linear or branched and optionally interrupted by one or more atoms or atom groups independently selected from the group consisting of -O-, -N(R²)-, - C(O)-N(R ²)- and optionally substituted with one or more substituents independently selected from the group consisting of, -OH and -N(R²)₂, wherein each R² is independently selected from the group consisting of H; and C₁₋₄ alkyl and wherein the chelating moiety within the C₁₋₂₀ alkyl chain comprises at least two carboxylic acid or carboxylate groups.

11. The contrast agent according to claim 10, wherein the chelating moiety comprises one or more amino polycarboxylic acids.

12. The contrast agent according to claim 11, wherein the amino polycarboxylic acid is independently selected from the group consisting of EDTA; DTPA; aspartic acid; and glutamic acid.

13. The contrast agent according to any of the preceding claims, wherein the first monomer unit is represented by the formula (I): 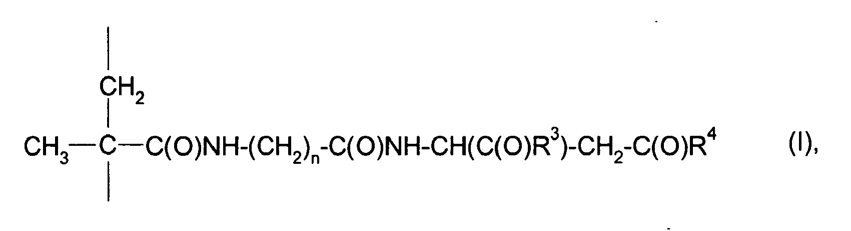 wherein n is an integer from 1 to 10 and R³, R⁴, are independently selected from the group consisting of OH; and -NH-CH(COOH)-CH₂-COOH).

14. The contrast agent according to any of the preceding claims, wherein the molar ratio of first monomer : second monomer unit is from 0,005 to 0,3.

15. The contrast agent according to any of the preceding claims comprising a copolymer of the formula (II) consisting of units of HPMA (second monomer) and HPMA derivative (first monomer) containing various amounts of the first monomer unit:

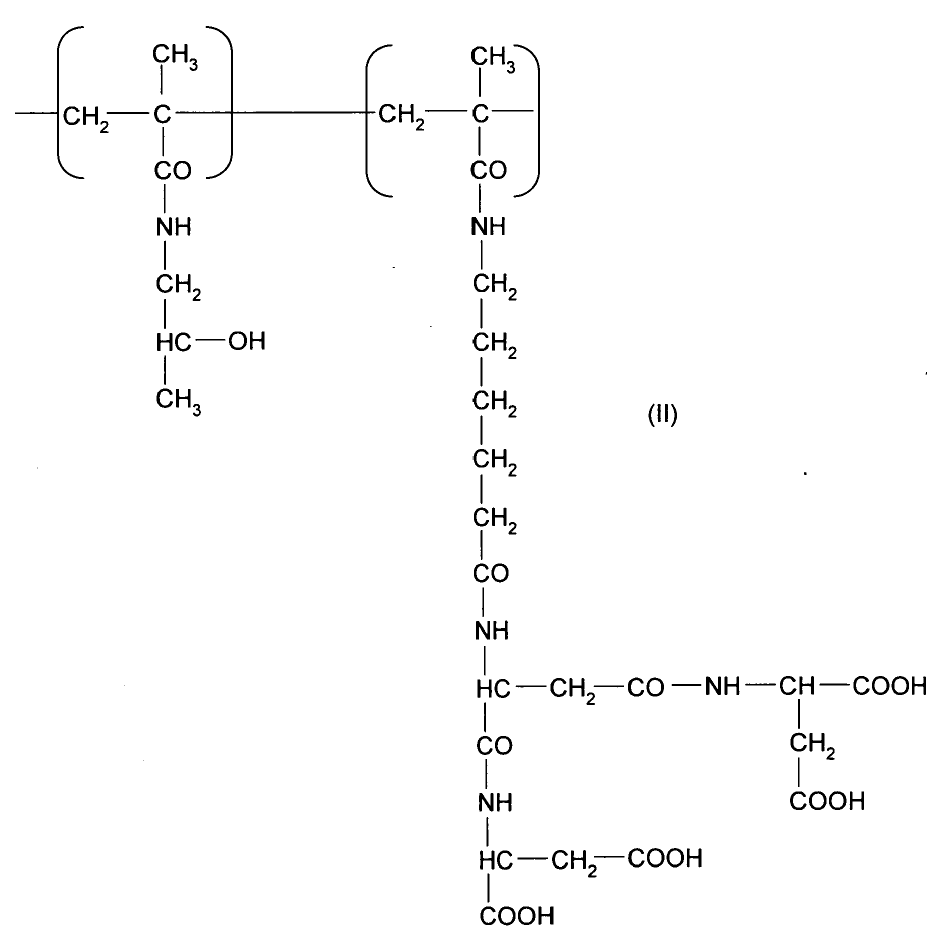

16. The contrast agent according to claim 15, wherein the copolymer contains from 0,5 mol-% to 30 mol-% of the first monomer unit.

17. The contrast agent according to any of the preceding claims, wherein the contrast enhancing metal is a paramagnetic metal ion.

18. The contrast agent according to claim 17, wherein the metal is selected from the group consisting of gadolinium, manganese, copper, chromium, iron, cobalt, erbium, nickel, europium and dysprosium.

19. A process for the preparation of a contrast agent according to any of the claims 1 to 18, comprising the steps:
a) polymerizing the first monomer with at least a second monomer in a solution, wherein functional groups of the monomers are optionally protected;
b) optionally deprotecting the formal copolymer;
c) adding the contrast enhancing metal as a salt to a copolymer solution; and
d) isolating the metal copolymer complex.

20. The process according to claim 19, wherein step a) is a radical solution or precipitation copolymerization.

21. The process according to claim 19 or 20, wherein carboxylic groups are protected in form of methyl or t.-butyl esters.

22. The process according to any of the claims 19 to 20, wherein the complex in step d) is isolated by dialysis and subsequent lyophilization.

23. Use of a contrast agent according to any of the claims 1 to 18 in magnetic resonance imaging (MRI), especially in MR angiography.
